# EUROPÄISCHE PATENTANMELDUNG

(11) **EP 1 997 377 A2**
(43) Veröffentlichungstag der Anmeldung: **03.12.2008**
(21) Anmeldenummer: 08007755.5
(22) Anmeldetag: 22.04.2008
(51) Int. Cl.: A01N 43/16, A01N 65/00, A01N 25/04, A61K 8/49, A61K 8/97, A61Q 17/02, A01P 17/00

(54) **Insektenschutzmittel mit reduzierter Klebrigkeit**

(30) Priorität: 31.05.2007 DE 102007026051
(71) Anmelder: Beiersdorf AG, 20245 Hamburg (DE)
(72) Erfinder: Schulz, Jens, Dr., 22869 Schenefeld (DE); Kröpke, Rainer, 22869 Schenefeld (DE); Lüttig, Kaja, 20255 Hamburg (DE); Fänger, Sabine, 22763 Hamburg (DE); von der Fecht, Stephanie, 22880 Wedel (DE); Nielsen, Jens, 24558 Henstedt-Ulzburg (DE)
(74) Vertreter: Hartmann, Jost

(57) **Zusammenfassung**

Die Erfindung beschreibt Insektenabwehrende Zubereitungen umfassend einen relativ hohen Anteil an einen oder mehreren Insektenrepellentwirkstoffen gewählt aus Dihydronepetalactonen und/oder Extrakten der Katzenminze und ein oder mehreren Füllstoffen mit einer spezifischen Oberfläche von mehr als 0,2 m²/g.

Die Zubereitungen zeichnen sich trotz des hohen Insektenrepellentgehaltes durch eine gute Hautverträglichkeit und verminderte Klebrigkeit aus.

## Beschreibung

Die vorliegende Erfindung betrifft kosmetische Formulierungen zur Insektenabwehr umfassend einen relativ hohen Anteil an Insektenrepellentwirkstoffen gewählt aus Dihydronepetalacton und/oder Extrakten der Katzenminze und Füllstoffen mit einer spezifischen Oberfläche von mindestens 0,2 m²/g zur Reduzierung der Klebrigkeit von kosmetischen Zubereitungen mit einem Gehalt an Dihydronepetalactone.

Die Kombination ermöglicht die Bereitstellung eines wirksamen Insektenschutzmittels mit gleichzeitig guter Hautverträglichkeit, verbesserter Sensorik sowie verminderter Klebrigkeit..

Insektenschutzmittel (Insektenvertreibungsmittel, Insektenabwehrmittel, Repellentien, Repellents) sind Präparate, die zur Abwehr und/oder Vertreibung von Insekten, aber auch Zecken und Milben äußerlich angewendet werden und verhindern sollen, dass diese auf der Haut aktiv werden. Insektenabwehrmittel sollen die Haut vor Belästigung durch Blut saugende oder beißende Insekten und andere Parasiten und/oder Lästlinge schützen, indem sie diese abwehren, bevor sie sich auf der Haut niederlassen. Die Mittel wirken dementsprechend nicht als Kontaktgifte, sondern nur als Abwehrmittel, da sie die Tiere nicht töten, sondern lediglich vertreiben.

Als Repellent-Wirkstoff ist beispielsweise 1-Piperidincarboxylsäure 2-(2-hydroxyethyl)-1-methylpropylester (INN: Icaridin, CAS-Nummer: 119515-38-7, Elincs-Nummer: 423-210-8) bekannt, der die folgende Struktur aufweist:

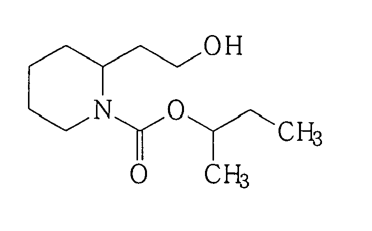

Ein weiterer eingesetzter Repellent-Wirkstoff ist der 3-(N-n-Butyl-N-acetyl-amino)propionsäureethylester (auch als Ethylbutylacetylaminopropionat oder Repellent 3535 bezeichnet), welcher sich durch die folgende Strukturformel auszeichnet:

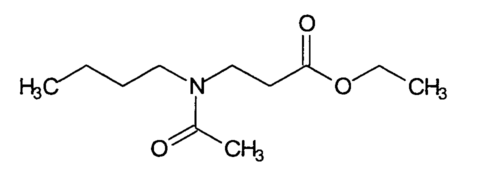

Nicht zuletzt kennt der Fachmann den Repellent-Wirkstoff N,N-Diethyl-3-methylbenzamid (Handelsbezeichnung: Meta-delphene, DEET), der die folgende Struktur aufweist:

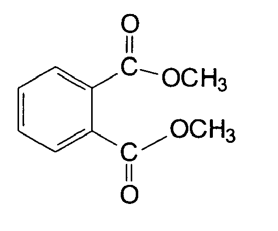

Diese Stoffe sind als Repellentwirkstoffe gegen Insekten, insbesondere Mücken oder Zecken, vielfach beschrieben, wie z.B. in DE 2246433, DE 19645250, DE 19645920.

In der WO 2006096876 und WO 2005034626 werden Dihydronepetalactone als Repellentien gegen Insekten offenbart. Der Offenbarungsgehalt zu Dihydronepetalacton der WO 2006096876 und WO 2005034626 sind hiermit explizit als erfindungsgemäß mitumfasst.

Katzenminze oder auch Nepeta ist als Mückenabwehrmittel bekannt und soll eine etwa zehn Mal stärkere Abwehr gegen Mücken als herkömmliche Abwehrmittel aufweisen.

In der DE 102005033845 werden des weiteren u.a bestimmte Parfuminhaltsstoffe zur Abwehr von Mücken, Sandfliegen, Läusen, Bremsen, Wespen, Bienen, Fliegen und Zecken erwähnt.

Ein Problem ist dabei, dass ein Repellentwirkstoff, der beispielsweise gegen Mücken abwehrend wirkt, diese Abwehrwirkung nicht oder nur eingeschränkt gegen andere Insekten aufweist.
Um wirksam insektenabwehrend zu wirken müssen die Repellentien zu einem relativ hohen Anteil zugesetzt werden. Um einen Vollschutz gegen Zecken oder auch der sehr aggressiven Anopheles Mücke zu erzielen, muss ein Zusatz von bis 20% des Repellents zugesetzt werden. Problem dabei ist, dass Zubereitungen mit einer hohen Konzentration an Repellentien bei Menschen mit besonders empfindlicher Haut in Einzelfällen zu Hautirritationen führen können. Ferner haben die Repellent-Wirkstoffe einen auch für die menschliche Nase wahrnehmbaren und auf Mitmenschen nicht gerade anziehend wirkenden Eigengeruch, der die Anwendung von Insektenabwehrmitteln nicht gerade attraktiv macht. Die abweisende Wirkung der Zubereitung erstreckt sich also nicht allein auf Insekten sondern darüber hinaus unter Umständen auch auf Mitmenschen, wenn diese über einen ausgeprägten Geruchsinn verfügen.

Die Zubereitungen des Standes der Technik haben bei derartig hohen Einsatzkonzentrationen den Nachteil, insbesondere bei Menschen mit empfindlicher oder zu Allergien neigender Haut, nicht besonders verträglich zu sein und in Einzelfällen zu Hautreizungen zu führen.

Wünschenswert ist es eine Zubereitung zur Verfügung zu stellen, die einerseits insektenabwehrend wirkt und andererseits hautverträglich gestaltet ist und vorteilhaft einen für die menschliche Nase angenehm wohlriechenden Duft verströmt.

Repellentien sind in der Regel ca. 2- 6 Stunden wirksam und verlieren danach aufgrund der Verdunstung ihre Wirksamkeit.

Es war daher die Aufgabe der vorliegenden Erfindung, kosmetische Zubereitungen mit einem Gehalt an Dihydronepetalactone zu entwickeln, die besonders hautfreundlich und hautverträglich sind, ein reduziertes Hautreizungspotential aufweisen und ein längeres Wirkpotential aufweisen.

Die Erfindung beschreibt eine Zubereitung umfassend einen oder mehrere Insektenrepellentwirkstoffe gewählt aus Dihydronepetalactonen und/oder Extrakten der Katzenminze und ein oder mehrere Füllstoffe mit einer spezifischen Oberfläche von mindestens 0,2 m²/g.

Die Zubereitungen lassen sich zur Reduzierung der Klebrigkeit von kosmetischen Zubereitungen, die Dihydronepetalactone enthalten, verwenden.

Insbesondere ist der Anteil an Repellentien in der Zubereitung relativ hoch, d.h. bis zu 40 Gew.% und bevorzugt im Bereich von 5 bis 20 Gew.%, bezogen auf die Gesamtmasse der Zubereitung.

Dihydronepetalactone sind beispielweise in der WO 2006096876 und WO 2005034626 beschrieben, die umfänglich zum Offenbarungsgehalt der vorliegenden Erfindung zählen.

Die Katzenminzen (Nepeta) bezeichnen eine Pflanzengattung der Familie der Lippenblütler (Lamiaceae). Erfindungsgemäß werden als Extrakte alle möglichen Extrakte, Lösungen oder Dispersionen der Nepetapflanze oder deren Teile verstanden.

Bei den in den Nepeta enthaltenen ätherischen Ölen handelt es sich in den Hauptbestandteilen um Citral, Citronellol, Geraniol und Limonen, sowie Nepetalacton, Gerb- und Bitterstoffe. Der Wirkstoff, der die Pflanze für Katzen so unwiderstehlich macht, ist Actinidin, ein iridoides Glykosid, das dem vergleichbaren Wirkstoff des Baldrian sehr ähnlich ist (BOWN, 1995). Die Nepeta enthält etwa 0,2-0,7 % ätherische Öle.

Der ölige Extrakt der Katzenminze, insbesondere aufgereinigt, ist wie jedes andere Naturöl sehr schlecht spreitend und sehr polar. Es ist deshalb unkosmetisch und verleiht den herkömmlichen kosmetischen Formeln eine ölig, fettige Phase. Es wird als schwer verteilbar wahrgenommen. Der polare Charakter des Extraktes führt zudem sehr schnell zu Instabilitäten, wie z.B. Öl- oder Wasserabscheidung.
Erfindungsgemäß ist diese Schwierigkeit der kosmetischen Formulierung durch die Kombination mit Füllstoffen mit einer spezifischen Oberfläche von mindestens 0,2 m²/g überwunden worden.

Die erfindungsgemäße Zubereitung kann die Dihydronepetalactone und/oder Extrakte der Katzenminze in unterschiedlichen Formen enthalten. Bevorzugt enthält die Zubereitung eine aufgereinigte Repellentienmischung, die zu etwa 90 Gew.% Dihydronepetalacton und 2 Gew.% Dehydronepetalacton, bezogen auf die Gesamtmischungsmasse, als wirksame Repellentien umfasst.

Bevorzugt ist eine erfindungsgemäße Zubereitung, die weiteren Insektenrepellentien, wie z.B. 1-Piperidincarboxylsäure 2-(2-hydroxyethyl)-1-methylpropylester (INN: Icaridin) und/oder N,N-Diethyl-3-methylbenzamid (DEET) und/oder Ethylbutylacetylaminopropionat (EBAAP) umfasst.

Zubereitungen, welche Dihydronepetalactone enthalten, haben den Nachteil, dass sie ein klebriges Hautgefühl beim und nach dem Auftragen auf der Haut hinterlassen, wenn der Wirkstoff DHPL in höheren Konzentrationen (5 Gew.% und mehr) eingesetzt wird.

Diese Klebrigkeit konnte durch den Zusatz von Füllstoffen mit einer spezifischen Oberfläche von mindestens 0,2 m²/g vermindert bzw. verhindert werden.

Erfindungsgemäß vorteilhaft sind ein oder mehrere Füllstoffe mit einer spezifischen Oberfläche von mehr als 0,2 m²/g zu einem Anteil von 0,5 bis 20 Gew.%, bevorzugt von 1 bis 10 Gew.%, jeweils bezogen auf das Gesamtgewicht der Zubereitung, enthalten.
Der oder die Füllstoffe werden vorteilhaft gewählt aus der Gruppe nativen Stärken, Nylon, Polyurethane, PMMA, Silikate, Talkum, Kaolin, Polythylene, PTFE, modifizierte Stärke und/oder Cellulose. Ganz besonders bevorzugt ist der Einsatz von Stärke, Talkum, Nylon sowie Polyethylen.

Die Kombination aus Dihydronepetalactonen und/oder Extrakten der Katzenminze und den Füllstoffen verlängert die Wirkungsdauer, da die Verdunstung der Repellentien verzögert wird. Erstaunlich ist darüber hinaus, dass die Sensorik der meist sich sehr klebrig auf der Haut anfühlenden Insektenabwehrmittel, insbesondere die Dihydronepetalacton enthaltenden, deutlich verbessert werden kann.

Bevorzugt sind Füllstoffe zu wählen aus der Gruppe der nativen Stärken, Nylon, Polyurethane, PMMA, Silikate, Talkum, Kaolin, Polythylene, PTFE, modifizierte Stärke und/oder Cellulose. Ganz besonders bevorzugt ist der Einsatz von Stärke, Talkum, Nylon sowie Polyethylen.die eine sehr gute Sensorikverbesserung hervorrufen.
Die erfindungsgemäße kosmetische Zubereitung führt zu einer Reduzierung der Klebrigkeit von kosmetischen Zubereitungen mit einem Gehalt an Dihydronepetalactonen.

Die Klebrigkeit wird oft subjektiv und damit unterschiedlich erlebt. Es wurden einige Formulierungen mittels der Deskriptive Sensorik von Lotionen nach der Spectrum Methode untersucht. Die Testung wird folgendermaßen durchgeführt:

Zunächst wird eine Beschreibung des Hautgefühls beim Auftragen und direkt nach dem Auftragen des Produktes durchgeführt.
Die Muster werden von trainierten Probanden mittels einer fest vorgebenen, standardisierten Vorgehensweise bewertet. Die Produkte werden dazu einmal auf den Unterarm appliziert und direkt bewertet.
Als Eichreferenz für die Probandinnen wird Nivea Body Lotion verwendet.
Nachstehend sind die Ergebnisse aufgeführt (je höher die Zahl, desto besser die Eigenschaft; Parameter auf einer Skala von 1 bis 10):

| **Parameter** | **Beispiel 40 ohne Nylon** | **Beispiel 40 ohne DHPL** | **Beispiel 40** |
|---|---|---|---|
| Verteilbarkeit | 4,5 | 6,5 | 7,5 |
| Einzugsvermögen | 6,5 | 8,5 | 8 |
| Klebrigkeit - sofort | 2,5 | 7,5 | 6 |
| Gleitfähigkeit - sofort | 5,5 | 7,5 | 6 |
| Rückstandsmenge - sofort | 5,5 | 8,5 | 7 |
| Klebrigkeit - 3min | 3,5 | 8 | 6 |
| Gleitfähigkeit - 3min | 4,5 | 7,5 | 6,5 |
| Rückstandsmenge - 3min | 5 | 8 | 6,5 |

Die Ergebnisse zeigen eine synergistische Verbesserung der Sensorikparameter wenn DHPL mit einem oder mehreren Füllstoffe (hier Nylon) mit einer spezifischen Oberfläche von mindestens 0,2 m²/g kombiniert wird.

Die spezifische Oberfläche der Füllstoffe kann mit bekannten und geeigneten Methoden, wie beispielsweise BET, bestimmt werden.

Neben der Insektenabwehr sind aber vor allem die Hautverträglichkeit und das Hautgefühl entscheidende Eigenschaften eines topisch zu applizierenden insbesondere kosmetischen Produktes.
Es nützt eine hervorragende Repellentwirksamkeit nichts, wenn das Kosmetikprodukt zu Unverträglichkeiten führt.
Eine der häufig auftretenden Hautunverträglichkeiten ist der sog. Stinging-Effekt, der sich insbesondere im Gesicht unangenehm bemerkbar macht. Der Stinging-Effekt tritt insbesondere im Nasolabial-Bereich auf und bedeutet ein subjektives Missempfinden der Probanden.

Um solche unerwünschten Effekte für Kosmetika möglichst auszuschliessen werden vor Produkteinführung sog. Stinging-Tests durchgeführt. Jeweils zwei Produkte werden dabei im dirketen Paarvergleich auf ihre Hautverträglichkeit getestet. Als interner High-Standard (Benchmark) wird dabei ein als hautverträglich bekanntes handelsübliches Produkt verglichen (NIVEA Visage Day Cream normal mixed).

In zahlreichen Untersuchungen wurde nun festgestellt, dass ein wesentlicher Beitrag zum Stinging durch die in vielen Kosmetika enthaltenen Parabene hervorgerufen wird, insbesondere wenn diese mit einem Anteil von mehr als 0,3 Gew.% in der Rezeptur vorliegen.
Parabene werden als Konservierungsmittel eingesetzt, so dass deren Ersatz mitunter schwierig ist.

Es wurde nun überraschenderweise festgestellt, dass die erfindungsgemäßen Zubereitungen sehr gute Repellentwirksamkeiten aufweisen und einen Stinging-Effekt nicht aufweisen.
Die erfindungsgemäßen Zubereitungen lassen sich daher bevorzugt mit einem Anteil von bis zu 0,3 Gew.% Konservierungsmittel, wie insbesondere Parabene, herstellen.
Bevorzugt kann auf den Zusatz von Konservierungsmitteln, wie z.B. Parabenen, ganz verzichtet werden.
D.h. es ist erstmals möglich Repellenthaltige Zubereitungen zur Verfügung zu stellen, die gleichzeitig verbesserte Verträglichkeit aufweisen und insbesondere keine Stinging-Effekte aufweisen.
Die erfindungsgemäßen Zubereitungen sind daher bevorzugt frei von bakteriziden-, fungiziden-, sporicidally d.h. sporenabtötenden, wirksamen Mitteln und/oder frei von Konservierungsmitteln. Weiterhin sind sie bevorzugt frei von Formaldehyd, Formaldehyddonatoren wie Diazolidinyl urea, Imidazolidinyl urea und/oder DMDM Hydantoin sowie insbesondere frei von Benzoesäure, p-Hydroxybenzoesäure und/oder oder deren Derivate oder Salze. Bevorzugt umfassen die erfindungsgemäßen Zubereitungen weniger als 0,3 Gew.% bzw. insbesondere sind sie frei von Parabenen, insbesondere frei von Methylparaben, Ethylparaben, Propylparaben, Isopropylparaben, Butylparaben, Isobutylparaben und/oder Benzylparaben. Auch 2,4-Dichlorobenzylalkohol, 4-Chloro-3,5-dimethyl-phenol, 2-Bromo-2-nitropropane-1,3-diol und/oder lodopropinylbutylcarbamate (IPBC) sowie Quaternium-15 (CAS 51229-78-8), Methyl-chloroisothiazolinone und/oder Methylisothiazolinon sind in den erfindungsgemäßen Zubereitungen nicht bzw. zu weniger als 0,3 Gew.% zu finden.
Die erfindungsgemäßen Zubereitungen sind besonders bevorzugt Parabenfrei.
Es bedeutet erfindungsgemäß "frei von" ein Anteil von weniger als 0,1 Gew.%, bezogen auf die Gesamtmasse der Zubereitung. Bevorzugt beträgt der Anteil an den zuvor genannten Mitteln und insbesondere Parabenen 0 Gew.%.

Mücken, Zecken und andere Insekten im Sinne der vorliegenden Offenbarung werden in erster Linie durch eine komplexe Duftmischung aus Milchsäure, Fettsäuren und Aminosäuren angelockt, die von der menschlichen Haut verströmt werden und den Menschen wie eine Duftglocke umgeben. Es ist nun ein Nachteil von Insektenabwehrmitteln des Standes der Technik, dass derartige Zubereitungen die natürliche, menschliche Duftkomposition nicht direkt maskieren, sondern ihr vielmehr eine zusätzliche, auf Insekten abstoßend wirkende Duftnote (die des Repellent) hinzufügen. Um das anziehende und abstoßende Reizpotential auf die Insekten über einen längeren Zeitraum zugunsten der abstoßenden Reizwirkung zu verschieben, ist es nach dem Stande der Technik notwendig, relativ große Mengen an Repellent-Wirkstoffen einzusetzen.

Die Repellent-Wirkstoffe haben einen auch für die menschliche Nase wahrnehmbaren und auf Mitmenschen nicht gerade anziehend wirkenden Eigengeruch, der die Anwendung von Insektenabwehrmitteln nicht gerade attraktiv macht.

Vorteilhaft werden der erfindungsgemäßen Zubereitung ein oder mehrere Parfuminhalststoffe zugesetzt, gewählt aus der Gruppe
- 1,3,4,6,7,8-Hexahydro-4,6,6,7,8,8-hexamethylcyclopenta-gamma-2-benzopyran
- 2,6-Dimethyl-7-octen-2-ol
- 2-Acetonapthone-1,2,3,4,5,6,7,8-octahydro-2,3,8,8-tetramethyl
- 2-Isobutyl-4-hydroxy-4-methyltetrahydropyran
- 2-tert-Pentylcyclohexylacetat
- 3,7-Dimethyl-2,6-octadien-1-ol
- 3-Methyl-5-phenyl-1-pentanol
- 7-Acetyl-1, 1,3,4,4,6-hexamethyltetralin
- Adipinsäurediester
- alpha-Amylcinnamaldehyd
- alpha-Isomethylionon
- Alpha-Methylionon
- Amyl C Butylphenylmethylpropionalcinnamal
- Amylcinnamylalkohol
- Amylsalicylat
- Anisalkohol
- Benzoin
- Benzylacetat
- Benzylalkohol
- Benzylbenzoat
- Benzylcinnamat
- Benzylsalicylat
- Bergamotöl
- bitteres Orangenöl
- Butylphenylmethylpropioal
- Cardamomöl
- Cedrol
- Cinnamal
- Cinnamylalkohol
- Citral
- Citronellol
- Citronellylmethylcrotonat
- Citronenöl
- Coumarin
- Diethylsuccinat
- d-Limonene
- Ethylenbrassylate
- Ethyllinalool
- Eugenol
- Evernia Furfuracea Extract
- Evernia Prunastri Extract
- Farnesol
- Geraniol
- Guajakholzöl
- Heliotropin
- Hexylcinnamal
- Hexylsalicylat
- Hydroxycitronellal
- Hydroxyisohexyl 3-Cyclohexencarboxaldehyde
- Isoeugenol
- Lavendelöl
- Lemonenöl
- Limonen
- Linalool
- Linalylacetat
- Mandarinenöl
- Menthyl PCA
- Methyl-2 Octynoat
- Methylbenzoat
- Methylcedrylketone
- Methyldihydrojasmonate
- Methylheptenon
- Muskatnussöl
- p-t-Butyl-alpha-methyldihydrocinnamic aldehyde
- Rosmarinöl
- süßes Orangenöl
- Terpineol
- Tonkabohnenöl
- Triethylcitrat und/oder
- Vanillin.

Vorteilhaft ist der Zusatz von Hexylsalicylat, Linalylacetat und/oder 2-Isobutyl-4-hydroxy-4-methyltetrahydropyran, die den Eigengeruch des Dihydronepetalactons besonders gut maskieren ohne einen anlockenden Effekt auf die Insekten auszuüben.

Die erfindungsgemäßen Parfuminhaltstoffe, ein oder mehrere, sind bevorzugt zu einer Gesamtkonzentration von 1 ppm bis 2,5 Gew.%, jeweils bezogen auf das Gesamtgewicht der Zubereitung, in dieser enthalten.

Eine vorteilhafte Ausführungsform im Sinne der vorliegenden Erfindung ist eine kosmetische Zubereitung als Hydrodispersionsgel enthaltend neben den Füllstroffen 15 Gew.% Dihydronepetalacton und 0,004 Gew.% Hexylsalicylat, jeweils bezogen auf das Gesamtgewicht der Zubereitung.

Die Zubereitung ist bevorzugt eine kosmetische Zubereitung und zum Auftragen auf die Haut geeignet.

Die erfindungsgemäße kosmetische Zubereitung ist vorteilhaft dadurch gekennzeichnet, das sie in Form einer wässrigen oder wässrig-alkoholischen Lösung, einer Emulsion (W/O, O/W, W/S, S/W oder multiple Emulsion, Makro- oder Mikroemulsion), einer Dispersion, einer Pickering-Emulsion, eines Gels, eines Hydrodispersionsgels oder einer wasserfreien Zubereitung vorliegt. Dabei ist es erfindungsgemäß bevorzugt, wenn die kosmetische Zubereitung in Form eines Hydrodispersionsgels vorliegt.

Die Zubereitung kann erfindungsgemäß auch in Form einer dünnflüssigen, sprühfähigen wässrigen oder wässrig-alkoholischen Lösung, in Form eines Gels, als Salbe, Creme oder Lotion (ggf. sprühbar) vorliegen.

Die Zubereitung kann erfindungsgemäß vorteilhaft auch als Spray oder als Tränkungsmedium für ein Pflaster oder Tuch eingesetzt werden. Daher sind auch Pflaster und Tücher getränkt mit der erfindungsgemäßen Zubereitung, erfindungsgemäß.

Zubereitungen mit Dihydronepetalacton als Wirkstoff können in Form von Lösungen, Emulsionen und Dispersionen hergestellt werden, bei denen der lipophile Wirkstoff von einer wässrigen Phase umgeben ist (z.B. O/W-Emulsion). Durch die Umhüllung des Wirkstoffs mit der wässrigen Phase wird dessen Freisetzung aber gebremst und die Zubereitung selbst ist bei der Anwendung auf der Haut länger wirksam gegen Mücken, Insekten, etc.

Nachteilig am Stande der Technik ist jedoch der Sachverhalt, das diese Zubereitungen aufgrund ihrer wässrigen äußeren Phase nicht besonders wasserfest sind und leicht von der Haut abgespült werden können. Ferner lassen sich in diesen Zubereitungen keine oxidationsempfindlichen, wasserlöslichen Zusatzstoffe einarbeiten.

Diese Mängel lassen sich erfindungsgemäß durch Emulsionen mit einer wässrigen inneren Phase und einer lipophilen äußeren Phase, enthaltend Dihydronepetalactone, beheben.

Besonders vorteilhaft liegen die erfindungsgemäßen Zubereitung daher in Form einer W/O- oder W/S-Emulsion vor.

Die erfindungsgemäßen Zubereitungen weisen dadurch eine verlängerte Wirkdauer und eine erhöhte Wasserfestigkeit auf der Haut auf.

Überraschend war insbesondere der Umstand, dass, obwohl der Repellent-Wirkstoff in der äußeren, lipophilen Phase angereichert ist, bei der Anwendung auf der Haut gleichmäßig über einen Zeitraum von mehreren Stunden an die Umwelt abgegeben wird und somit eine lang anhaltende Repellent-Wirkung entfaltet.

Überraschend war nicht zuletzt, dass bei den erfindungsgemäßen Zubereitungen die Klebrigkeit der Zubereitung auf der Haut im Vergleich zu Produkten des Stand der Technik (mit gleichem Repellent-Gehalt) deutlich reduziert ist.

Die Wasserphase der erfindungsgemäßen Zubereitung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Eine eventuell vorhandene Ölphase kann alle herkömmlichen Bestandteile von in der Kosmetik eingesetzten Öl-, Fett- und Wachskomponenten enthalten.

Als Emulgatoren können alle aus der Kosmetik bekannten Emulgatoren und Emulgatorsysteme eingesetzt werden.

Neben der erfindungsgemäßen Wirkstoff-Kombination kann die erfindungsgemäße Zubereitung weitere kosmetische Wirk- und Pflegestoffe enthalten, beispielsweise die nach der Kosmetikverordnung zugelassenen UV-Lichtschutzfilter, Konservierungsmittel bzw. Konservierungshelfer. Derartige Wirkstoffe können erfindungsgemäß vorteilhaft in Konzentrationen von 0,01 bis 30 Gew-%, bezogen auf das Gesamtgewicht der Zubereitung in dieser enthalten sein.

Als weitere Pflegestoffe können insbesondere Niacinamid, Panthenol, Aloe vera, Hammamelis-Extrakt, Polidocanol, Vitamin E, Vitamin E-Derivate, Vitamin A, Vitamin A-Derivate, Vitamin C, Vitamin C-Derivate, Coenzym Q10, Kreatin, Taurin, Alpha-Glycosylrutin in Konzentrationen von 0,1 bis 30 Gewichts-%, bezogen auf das Gesamtgewicht der Zubereitung eingesetzt werden.

Erfindungsgemäß besonders bevorzugt enthält die erfindungsgemäße Zubereitung als weitere Bestandteile Alpha-Hydroxysäuren und/oder deren Salze. Dabei werden erfindungsgemäß bevorzugt Milchsäure/Lactate oder Zitronensäure/Citrate in einer Konzentration von 0,01 bis 5 Gewichts-%, bezogen auf das Gesamtgewicht der Zubereitung eingesetzt.

Als insbesondere kosmetische Zubereitung ist die erfindungsgemäße Zubereitung als topisch anzuwendendes Mittel vorteilhaft geeignet.

Ebenso ist die Verwendung einer erfindungsgemäßen Zubereitung als Insektenabwehrmittel in einer Verdunstungsvorrichtung möglich und bevorzugt.

Die nachfolgenden Beispiele sollen die vorliegende Erfindung verdeutlichen, ohne sie einzuschränken. Die Angaben beziehen sich stets auf Gewichts-%, sofern nicht andere Angaben gemacht werden.

### W/O-Emulsionen

| | **1** | **2** | **3** | **4** | **5** |
|---|---|---|---|---|---|
| Triglycerindiisostearat | 1,0 | 0,5 | 0,25 | 2,0 | 3,0 |
| Diglycerindipolyhydroxystearat | 1,0 | 1,5 | 1,75 | 3,0 | 2,0 |
| Paraffinöl | 12,5 | 10,0 | 8,0 | 5,0 | 7,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Decyloleat | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Cellulose | 0,5 | 1,0 | --- | 0,25 | 2,5 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 0,2 | 0.05 | 0.4 | 0.3 | 2,0 |
| Parfum | q,s, | q,s, | q,s, | q,s, | q,s, |
| Ethanol | 2,0 | --- | 5,0 | --- | --- |
| Capryl-/Caprinsäuretriglycerid | 2,0 | 2,5 | 3,0 | 5,0 | 0,5 |
| Kaliumsorbat | 0,04 | 0,15 | 0,05 | 0,03 | 0,4 |
| Benzylalkohol | 0,3 | 0,4 | 0,25 | 0,15 | --- |
| PTFE | --- | --- | 1,5 | --- | --- |
| Dihydronepetalacton | 5 | 7,5 | 10 | 12,5 | 30 |
| Wasser | ad 100 | ad 100 | ad 100 | ad 100 | ad 100 |

### W/O-Emulsionen

| | **6** | **7** | **8** | **9** | **10** |
|---|---|---|---|---|---|
| PEG-30 Dipolyhydroxystearat | --- | 0,5 | 0,25 | --- | 8,0 |
| Wollwachsalkohol | 1,0 | 1,5 | 1,75 | 3,0 | -- |
| Paraffinöl | 12,5 | 10,0 | 8,0 | 5,0 | 12,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Hydriertes Polyisobuten | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Microcrystalline Cellulose | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Hydroxycitronellal | 0,05 | 0,1 | 0,0125 | 0,75 | 0,0375 |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Natriumdehydracet | --- | --- | 0,05 | --- | --- |
| Kaliumsorbat | 0,3 | 0,4 | 0,25 | 0,15 | --- |
| Polyurethan | 1,5 | --- | 1,0 | 2,5 | 10 |
| Dihydronepetalacton | 7,5 | 12,5 | 17,5 | 20 | 35 |
| Talkum | 1,5 | 3,5 | --- | --- | --- |
| Wasser | ad 100 | ad 100 | ad 100 | ad 100 | ad 100 |

### W/S-Emulsion

| | **11** | **12** | **13** | **14** | **15** |
|---|---|---|---|---|---|
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| Cyclomethicon | 12,5 | 15 | 28,0 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 1,0 | 0,1 | 0,4 | 0,9 | 2,5 |
| Parfum | q,s, | q,s, | q,s, | q,s, | q,s, |
| Kaliumsorbat | 0,4 | 0,1 | 0,05 | 0,3 | 0,4 |
| Cetyldimethicon | 0,5 | --- | 0,7 | --- | --- |
| Benzylalkohol | --- | --- | 0,05 | --- | 0,1 |
| Polyethylen | 0,3 | 6 | 1,25 | 2,0 | 7,5 |
| Dihydronepetalacton | 3 | 15 | 12,5 | 20 | 7,5 |
| Wasser | ad 100 | ad 100 | ad 100 | ad 100 | ad 100 |

### W/S-Emulsionen

| | **16** | **17** | **18** | **19** | **20** |
|---|---|---|---|---|---|
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| Cyclomethicon | 12,5 | 15 | 28,0 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Milchsäure | 0,2 | 0,1 | 0,2 | --- | --- |
| Natriumlactat | 0,2 | 1,0 | 0.05 | --- | --- |
| Parfum | q,s, | q,s, | q,s, | q,s, | q,s, |
| Dihydronepetalacton | 3,0 | 10 | 12,5 | 15 | 30 |
| Stearyldimethicon | 0,5 | --- | 0,7 | --- | --- |
| Dehydracetsäure | --- | --- | 0,05 | --- | 0,1 |
| Tapiocastärke | 0,3 | 2,0 | 12,5 | 1,5 | 3 |
| Wasser | ad 100 | ad 100 | ad 100 | ad 100 | ad 100 |

### W/O-Emulsionen-parabenfrei

| | **21** | **22** | **23** | **24** | **25** |
|---|---|---|---|---|---|
| PEG-22 Dodecyl Glycol Copolymer | 5,0 | 1,5 | 0,25 | --- | 3,0 |
| PEG-45 Dodecyl Glycol Polymer | 1,0 | 1,5 | 1,75 | 3,0 | -- |
| Paraffinöl | 12,5 | 10,0 | 8,0 | 5,0 | 17,5 |
| Isopropylstearat | 8,0 | 6,0 | 5,0 | 12,0 | 2,5 |
| hydrierte Kokosglyceride | 2,0 | 1,0 | 2,5 | 5,0 | 0,25 |
| Nachtkerzenöl | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Talkum | 0,5 | 4 | 1,5 | 2,0 | 10 |
| Dihydronepetalacton und Katzenminzeextrakt | 5 | 10 | 15 | 20 | 25 |
| Magnesiumsulfat | 0,5 | 0,6 | 0,5 | 0,7 | 1,0 |
| Glycerin | 3,0 | 5,0 | 10,0 | 15,0 | 1,5 |
| Natriumcitrat | 0,2 | 0,1 | --- | --- | --- |
| Zitronensäure | 0,2 | 0,1 | --- | --- | --- |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Kaliumsorbat | 0,4 | 0,15 | 0,05 | 0,3 | 0,4 |
| Benzylalkohol | --- | --- | 0,05 | --- | 0,1 |
| Wasser | ad 100 | ad 100 | ad 100 | ad 100 | ad 100 |

### W/O-Emulsionen

| | **26** | **27** | **28** | **29** | **30** |
|---|---|---|---|---|---|
| Polyglyceryl-2-dipolyhydroxystearat | 3,0 | --- | 0,25 | --- | 6,0 |
| Polyglyceryl-3 diisostearate | 1,0 | 3,5 | 1,75 | 2,5 | -- |
| PEG-40 sorbitanisostearate | --- | 2,5 | 0,5 | 3,5 | 3,0 |
| Paraffinöl | 12,5 | 10,0 | 8,0 | 5,0 | 17,5 |
| Isopropylstearat | 8,0 | 6,0 | 5,0 | 12,0 | 2,5 |
| hydrierte Kokosglyceride | 2,0 | 1,0 | 2,5 | 5,0 | 0,25 |
| Isopropylpalmitat | 0,5 | 1,0 | 0,75 | 3,0 | 0,25 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Cellulose | 1,618 | 1,618 | 0,75 | 1,5 | 4 |
| Dihydronepetalacton | 1,618 | 16,18 | 7,5 | 15 | 40 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,1 | 0,3 | 1,0 |
| Natriumcitrat | 0,2 | 0,3 | 0,2 | 1,5 | 0.8 |
| Parfum | q,s, | q,s, | q,s, | q,s, | q,s, |
| Capryl-/Caprinsäuretriglycerid | 2,0 | 2,5 | 3,0 | 5,0 | 0,5 |
| Kaliumsorbat | 0,24 | 0,15 | 0,05 | 0,3 | 0,4 |
| Wasser | ad 100 | ad 100 | ad 100 | ad 100 | ad 100 |

### Silikon in Wasser-Emulsion

| | **31** | **32** | **33** | **34** | **35** |
|---|---|---|---|---|---|
| Dimethiconcopolyol, | 1,0 | 2,0 | 8,0 | 3,0 | 5,0 |
| Caprylic/Caprictriglycerid | | | | | |
| Cyclomethicon | 12,5 | 15 | 25,0 | 10,0 | 7,5 |
| Dimethicon | 5,0 | 15,0 | 5,0 | 12,0 | 15,0 |
| Mineralöl | 0,5 | 0,75 | 1,0 | 2,0 | 0,25 |
| Phenyltrimethicon | 0,5 | 1,0 | 0,75 | 3,0 | 0,25 |
| Glycerin | 5,0 | 7,5 | 10,0 | 3,0 | 1,0 |
| Talkum | 1 | 4 | 8 | 4 | 1,5 |
| Dihydronepetalacton | 10 | 20 | 30 | 40 | 15 |
| Xanthan Gum | --- | 0,1 | -- | 0,25 | 1,0 |
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| Methylisothiazolinone | --- | --- | 0,05 | --- | 0,1 |
| Wasser | ad 100 | ad 100 | ad 100 | ad 100 | ad 100 |

### O/W-Emulsion-parabenfrei

| | **36** | **37** | **38** | **39** | **40** |
|---|---|---|---|---|---|
| Glycerylsterat | 1,0 | --- | --- | 0,5 | 0,75 |
| Polyethylenglycol(40)stearat | 10,0 | --- | 5 | --- | --- |
| Triglycerinmethylglucosedistearat | --- | 5,5 | --- | --- | 3,5 |
| Sorbitanstearat | --- | 1,5 | 3 | --- | --- |
| Cyclomethicon | 2,5 | 15 | 8,0 | 5,0 | 7,5 |
| Dimethicon | 5,0 | 3,0 | 5,0 | 2,0 | 5,0 |
| Behenylalkohol | 1 | --- | 2 | 1 | --- |
| Stearylalkohol | --- | 1 | --- | 1 | --- |
| Ginkgo Biloba | 0,1 | 0,5 | 1,0 | 2,5 | 1,0 |
| Dihydronepetalacton | 7,5 | 15 | 12,5 | 20 | 25 |
| Cetylstearylalkohol | --- | --- | 1 | 1 | --- |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Parfum | q,s, | q,s, | q,s, | q,s, | q,s, |
| Kaliumsorbat | 0,4 | 0,1 | 0,05 | 0,3 | 0,4 |
| Methylisothiazolinone | --- | --- | 0,05 | --- | 0,1 |
| Glycerin | 5 | 10 | 3 | 15 | 7,5 |
| Nylon | 0,75 | 1,5 | 5 | 2,0 | 5 |
| Wasser | ad 100 | ad 100 | ad 100 | ad 100 | ad 100 |

### O/W-Emulsion

| | **41** | **42** | **43** | **44** | **45** |
|---|---|---|---|---|---|
| Polyethylenglycol(21)stearylether | 1 | --- | 2,5 | 2 | 1,5 |
| Polyethylenglycol(2)stearylether | 1 | --- | 5,5 | 3 | 7,5 |
| Cetearylglucosid | --- | 8 | --- | --- | --- |
| Behenylalkohol | 3 | 2 | --- | 1 | --- |
| Stearylalkohol | 3 | 2 | --- | 2 | --- |
| Cetylstearylalkohol | 3 | 4 | --- | --- | 2 |
| hydriertes Polyisobuten | 0,5 | 0,75 | 1,0 | 2,0 | 0,25 |
| Katzenminzeextrakt | 10 | 20 | 35 | 7,5 | 5,0 |
| Octyldodecanol | 0,5 | 1,0 | 0,75 | 3,0 | 0,25 |
| Glycerin | 5 | 10 | 15 | 3 | 7,5 |
| Panthenol | 0,5 | 1,0 | 0,75 | 0,25 | 0,1 |
| Citronellol | 0,25 | 0,0125 | 0,1 | 0,0005 | 0,85 |
| Methylparaben | 0,4 | 0,1 | 0,05 | 0,3 | 0,4 |
| Propylparaben | 0,3 | 0,4 | 0,25 | 0,15 | --- |
| Methylisothiazolinone | --- | --- | 0,05 | --- | 0,1 |
| Silikat | 1 | 2 | 1,5 | 3 | 0,5 |
| Wasser | ad 100 | ad 100 | ad 100 | ad 100 | ad 100 |

### O/W-Emulsion

| | **46** | **47** | **48** | **49** | **50** |
|---|---|---|---|---|---|
| Glycerylsteratcitrat | 1,0 | 0,5 | 1,5 | 9,0 | 0,3 |
| Polyethylenglycol(20)cetearylether | 10,0 | 1,0 | 5 | --- | --- |
| Triglycerinmethylglucosedistearat | --- | --- | 3,5 | --- | 2,5 |
| PMMA | 0,75 | 1,5 | 2,25 | 12 | 3,75 |
| Dihydronepetalacton | 7,5 | 15 | 22,5 | 30 | 37,5 |
| Dimethicon | 0,5 | 3,0 | 0,75 | 1,5 | 0,2 |
| Behenylalkohol | 1 | --- | 2 | --- | 0,2 |
| Dicaprylylcarbonat | 3 | 5 | 10 | 5 | 5 |
| Stearylalkohol | --- | --- | --- | 1 | 0,2 |
| Cetylstearylalkohol | --- | --- | 1 | 1 | 0,2 |
| Tocopherol | 0,5 | 0,5 | 0,75 | 0,25 | 0,1 |
| Octyldodecanol | 0,5 | --- | 0,75 | 3,0 | 0,25 |
| Panthenol | 0,5 | --- | 0,75 | 0,25 | 0,1 |
| Carbomer | 0,05 | 0,35 | 0,15 | 0,1 | --- |
| Parfum | q,s, | q,s, | q,s, | q,s, | q,s, |
| Caprylic/Capric Triglycerid | 1 | 5 | 3 | 5 | 10 |
| Methylparaben | 0,4 | 0,3 | 0,05 | 0,3 | 0,4 |
| Propylparaben | 0,3 | --- | 0,25 | 0,15 | --- |
| Methylisothiazolinone | --- | --- | 0,05 | --- | 0,1 |
| Phenoxyethanol | --- | 0,5 | --- | 0,15 | --- |
| Sorbitol | 10 | --- | --- | 5 | --- |
| Butylenglykol | --- | --- | --- | 5 | 10 |
| Propylenglykol | --- | --- | 10 | 5 | --- |
| Glycerin | --- | 7,5 | --- | --- | --- |
| Wasser | ad 100 | ad 100 | ad 100 | ad 100 | ad 100 |

### O/W-Emulsion

| | **51** | **52** | **53** | **54** | **55** |
|---|---|---|---|---|---|
| Glycerylsterat | 2,6 | --- | --- | 2,7 | 2,6 |
| Caprylic/Capric Triglycerid | 3,5 | 4,0 | 3,75 | 3,1 | 5,5 |
| Dicaprylylcarbonat | 3,5 | 2,5 | 3,75 | --- | 3,0 |
| Triglycerinmethylglucosedistearat | --- | 3,0 | 3,0 | --- | --- |
| Cetystearylalkohol | 3,0 | --- | --- | 4,0 | 3,5 |
| Dimethicon | --- | 3,0 | 5,0 | 3,0 | 2,75 |
| Cetylalkohol | --- | 3,0 | 1,5 | 1,25 | --- |
| Polyethylenglycol(40)stearat | 0,8 | --- | --- | 1,3 | 1,3 |
| C12-15 Alkylbenzoat | --- | 2,5 | 3,75 | 3,0 | 4,5 |
| PTFE | 0,5 | 1,0 | 1,5 | 4 | 2,5 |
| Dihydronepetalacton | 5 | 10 | 15 | 20 | 25 |
| Natriumoleanolat | 0,1 | 0,1 | 0,1 | 0,075 | 0,1 |
| Cyclomethicon | 3,5 | --- | --- | --- | 0,25 |
| Propylenglykol | 0,5 | 0,5 | 0,5 | 0,35 | 0,2 |
| Mineralöl | --- | --- | --- | 6,0 | --- |
| Phenoxyethanol | 0,4 | 0,4 | 0,4 | 0,75 | 1,25 |
| Zitronensäure | --- | --- | 0,05 | --- | --- |
| Ethanol | --- | --- | --- | 3,0 | --- |
| Glycerin | 10,0 | 10,0 | 10,0 | 10,0 | 12,5 |
| Carbomer, Natrium-Salz | 0,1 | 0,1 | 0,1 | 0,2 | 0,1 |
| Parfum | q,s, | q,s, | q,s, | q,s, | q,s, |
| Methylisothiazolinone | 0,06 | 0,08 | 0,04 | 0,1 | 0,07 |
| Wasser | ad 100 | ad 100 | ad 100 | ad 100 | ad 100 |

### Pumpspray

| | **56** | **57** | **58** | **59** | **60** |
|---|---|---|---|---|---|
| Polyethylenglycol(40)stearat | 1,0 | --- | 0,5 | --- | --- |
| Cyclomethicon | 0,5 | --- | --- | --- | --- |
| Dihydronepetalacton | 2 | 10 | 15 | 20 | 40 |
| Parfum | 0,15 | 0,25 | 0,35 | 1,0 | 0,4 |
| Ethanol | 15 | 20 | 30 | 40 | 20 |
| Methylparaben | 0,4 | 0,1 | 0,05 | 0,3 | 0,4 |
| Meeresextrakt | --- | --- | 0,1 | --- | --- |
| Aloe Vera Extrakt | 0,1 | --- | 0,1 | --- | --- |
| Hammamelis-Extrakt | 0,1 | --- | 0,1 | 5 | 0,5 |
| Glycerin | 5 | 10 | 3 | 15 | 7,5 |
| Talkum | 1,0 | 2,5 | 3,5 | 0,15 | 7,5 |
| Wasser | ad 100 | ad 100 | ad 100 | ad 100 | ad 100 |

### Spray mit Repellent

| | **61** | **62** | **63** | **64** | **65** |
|---|---|---|---|---|---|
| Ethylbutylacetylaminopropionat | 5 | 35 | 15 | 20 | 7,5 |
| N, N-Diethyltoluolamid | 5 | --- | --- | 1 | --- |
| Dihydronepetalacton | 5 | 3,0 | 2,0 | 1,5 | 12,5 |
| 1-(3-Cyclohexen-1-yl-carbonyl)-2-methylpiperidin | 5 | --- | 2 | 1 | --- |
| Hammamelis-Extrakt | 0,2 | --- | --- | 1 | 0,2 |
| Aloe Vera Extrakt | --- | --- | 1 | 1 | 5,0 |
| Tocopherol | 0,5 | 0,5 | 0,75 | 0,25 | 0,1 |
| Meeresextrakt | 0,5 | --- | 0,75 | 3,0 | 0,25 |
| Panthenol | 0,5 | --- | 0,75 | 0,25 | 0,1 |
| Nylon | 1 | 1,5 | 0,5 | 0,75 | 10 |
| Parfum | q,s, | q,s, | q,s, | q,s, | q,s, |
| Sorbitol | 10 | --- | --- | 5 | --- |
| Butylenglykol | --- | --- | --- | 5 | 10 |
| Propylenglykol | --- | --- | 10 | 5 | --- |
| Glycerin | --- | 7,5 | --- | --- | --- |
| Wasser | ad 100 | ad 100 | ad 100 | ad 100 | ad 100 |

## Patentansprüche

1. Zubereitung umfassend einen oder mehrere Insektenrepellentwirkstoffe gewählt aus Dihydronepetalactonen und/oder Extrakten der Katzenminze und ein oder mehrere Füllstoffe mit einer spezifischen Oberfläche von mindestens 0,2 m²/g.

2. Zubereitung nach Anspruch 1, **dadurch gekennzeichnet, dass** der Anteil an Repellentien 5 Gew% und mehr umfasst, bezogen auf die Gesamtmasse der Zubereitung.

3. Zubereitung nach einem der vorstehenden Ansprüche, **dadurch gekennzeichnet, dass** die Zubereitung eine aufgereinigte Repellentienmischung, die zu etwa 90 Gew.% Dihydronepetalacton und 2 Gew.% Dehydronepetalacton umfasst, bezogen auf die Mischungsmasse, enthält.

4. Zubereitung nach einem der vorstehenden Ansprüche, **dadurch gekennzeichnet, dass** die Zubereitung ein oder mehrere weitere Insektenrepellentien enthält.

5. Zubereitung nach einem der vorstehenden Ansprüche, **dadurch gekennzeichnet, dass** die Füllstoffe gewählt werden aus der Gruppe der nativen Stärken, Nylon, Polyurethan, PMMA, Silikate, Talkum, Kaolin, Polythylene, PTFE, modifizierte Stärke und/oder Cellulose.

6. Zubereitung nach einem der vorstehenden Ansprüche, **dadurch gekennzeichnet, dass** die Füllstoffe zu einem Anteil von 0,5 bis 20 Gew.%, bevorzugt von 1 bis 10 Gew.%, jeweils bezogen auf das Gesamtgewicht der Zubereitung, enthalten sind.

7. Zubereitung nach einem der vorstehenden Ansprüche, **dadurch gekennzeichnet,dass** die Zubereitung ein oder mehrere Parfuminhaltsstoffe gewählt aus der Gruppe 1,3,4,6,7,8-Hexahydro-4,6,6,7,8,8-hexamethylcyclopenta-gamma-2-benzopyran, 2,6-Dimethyl-7-octen-2-ol, 2-Acetonapthone-1,2,3,4,5,6,7,8-octahydro-2,3,8,8-tetramethyl, 2-Isobutyl-4-hydroxy-4-methyltetrahydropyran, 2-tert-Pentylcyclohexylacetat, 3,7-Dimethyl-2,6-octadien-1-ol, 3-Methyl-5-phenyl-1-pentanol, 7-Acetyl-1,1,3,4,4,6-hexamethyltetralin, Adipinsäurediester, alpha-Amylcinnamaldehyd, alpha-Isomethylionon, Alpha-Methylionon, Amyl C Butylphenylmethylpropionalcinnamal, Amylcinnamylalkohol, Amylsalicylat, Anisalkohol, Benzoin, Benzylacetat, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citral, Citronellol, Citronellylmethylcrotonat, Citronenöl, Coumarin, Diethylsuccinat, d-Limonene, Ethylenbrassylate, Ethyllinalool, Eugenol, Evernia Furfuracea Extract, Evernia Prunastri Extract, Farnesol, Geraniol, Guajakholzöl, Heliotropin, Hexylcinnamal, Hexylsalicylat, Hydroxycitronellal, Hydroxyisohexyl 3-Cyclohexencarboxaldehyde, Isoeugenol, Lavendelöl, Lemonenöl, Limonen, Linalool, Linalylacetat, Mandarinenöl, Menthyl PCA, Methyl-2 Octynoat, Methylbenzoat, Methylcedrylketone, Methyldihydrojasmonate, Methylheptenon, Muskatnussöl, p-t-Butyl-alpha-methyldihydrocinnamic aldehyde, Rosmarinöl, süßes Orangenöl, Terpineol, Tonkabohnenöl, Triethylcitrat und/oder Vanillin umfasst.

8. Zubereitung nach einem der vorstehenden Ansprüche, **dadurch gekennzeichnet, dass** die Zubereitung ein oder mehrere Parfuminhaltsstoffe gewählt aus der Gruppe Hexylsalicylat, Linalylacetat und/oder 2-Isobutyl-4-hydroxy-4-methyltetrahydropyran umfasst.

9. Zubereitung nach einem der vorstehenden Ansprüche, **dadurch gekennzeichnet, dass** die Zubereitung maximal 0,3 Gew.% bakterizide-, fungizide-, sporicidally wirksame Mittel, Konservierungsmittel und/oder Formaldehyd und/oder Formaldehyddonatoren enthält.

10. Zubereitung nach einem der vorstehenden Ansprüche, **dadurch gekennzeichnet, dass** die Zubereitung frei von bakteriziden-, fungiziden-, sporicidally wirksamen Mitteln, Konservierungsmitteln und/oder Formaldehyd und/oder Formaldehyddonatoren ist.

11. Zubereitung nach einem der vorstehenden Ansprüche, **dadurch gekennzeichnet, dass** die Zubereitung frei von Parabenen ist.

12. Zubereitung nach einem der vorstehenden Ansprüche in Form eines Hydrodispersionsgel.

13. Zubereitung nach einem der vorstehenden Ansprüche in Form einer W/O- oder W/S-Emulsion.

14. Zubereitung nach einem der vorstehenden Ansprüche 1 bis 12 in Form einer O/W- oder S/W-Emulsion.

15. Verwendung einer Zubereitung nach einem der vorstehenden Ansprüche als Insektenabwehrmittel.

16. Verwendung einer Zubereitung nach einem der Ansprüche 1 bis 14 zur Reduzierung der Klebrigkeit von kosmetischen Zubereitungen mit einem Gehalt an Dihydronepetalactonen.

17. Verwendung einer Zubereitung nach einem der Ansprüche 1 bis 14 als Insektenabwehrmittel in einer Verdunstungsvorrichtung.

18. Verwendung einer Zubereitung nach einem der vorstehenden Ansprüche 1 bis 14, **dadurch gekennzeichnet, dass** die Zubereitung als Tränkungsmedium auf Vlies, Pflaster oder Tuch aufgetragen wird.
